# EUROPEAN PATENT SPECIFICATION

(11) **EP 2 138 580 B1**
(45) Date of publication and mention of the grant of the patent: **20.03.2013**
(21) Application number: 08740493.5
(22) Date of filing: 16.04.2008
(51) Int. Cl.: C12N 15/09, A61K 35/76, A61K 38/46, A61K 48/00, A61P 43/00, C12N 5/10, C12N 9/22

(54) **VECTOR FOR GENE THERAPY**
VEKTOR FÜR GENTHERAPIE
VECTEUR POUR UNE THÉRAPIE GÉNIQUE

(30) Priority: 20.04.2007 JP 2007112178; 25.05.2007 JP 2007138889; 15.10.2007 JP 2007268310; 05.02.2008 JP 2008025248
(43) Date of publication of application: 30.12.2009
(73) Proprietor: Takara Bio, Inc., Otsu-shi Shiga 520-2193 (JP)
(72) Inventor: CHONO, Hideto, Otsu-shi Shiga 520-2193 (JP); MATSUMOTO, Kazuya, Otsu-shi Shiga 520-2193 (JP); MATSUMURA, Hajime, Otsu-shi Shiga 520-2193 (JP); MINENO, Junichi, Otsu-shi Shiga 520-2193 (JP); KATO, Ikunoshin, Otsu-shi Shiga 520-2193 (JP)
(74) Representative: Müller, Christian Stefan Gerd
(86) International application number: PCT/JP2008/057414
(87) International publication number: WO 2008/133137

(56) References cited:
- WO-A1-2007/020873
- JP-A- 2005 510 455
- JP-A- 2007 503 218
- MINENO JUNICHI ET AL: "Development of anti-HIV gene therapy strategy using novel endoribonuclease, MazF (II)", JOURNAL OF GENE MEDICINE, WILEY, US LNKD- DOI:10.1002/JGM.988, vol. 8, no. 12, 1 December 2006 (2006-12-01), page 1465, XP009122982, ISSN: 1099-498X [retrieved on 2006-11-27]
- JOSHI P J ET AL: "Aptamers directed to HIV-1 reverse transcriptase display greater efficacy over small hairpin RNAs targeted to viral RNA in blocking HIV-1 replication", MOLECULAR THERAPY, ACADEMIC PRESS, SAN DIEGO, CA, US LNKD- DOI:10.1016/J.YMTHE.2005.01.013, vol. 11, no. 5, 1 May 2005 (2005-05-01), pages 677-686, XP004863175, ISSN: 1525-0016
- BURKE JOHN DOUGLAS ET AL: "Retroviral vectors encoding a reverse transcription-activated transgene efficiently limit expression of the gene to target cells.", MOLECULAR THERAPY : THE JOURNAL OF THE AMERICAN SOCIETY OF GENE THERAPY MAR 2007 LNKD- PUBMED:17191073, vol. 15, no. 3, March 2007 (2007-03), pages 552-559, XP002609190, ISSN: 1525-0024
- BRADY H J M ET AL: "SPECIFIC ABLATION OF HUMAN IMMUNODEFICIENCY VIRYS TAT-EXPRESSING CELLS BY CONDITIONALLY TOXIC RETROVIRUSES", PROCEEDINGS OF THE NATIONAL ACADEMY OF SCIENCES OF THE UNITED STATES (PNAS), NATIONAL ACADEMY OF SCIENCE, US LNKD- DOI:10.1073/PNAS.91.1.365, vol. 91, 4 January 1994 (1994-01-04), pages 365-369, XP002062302, ISSN: 0027-8424
- Chono, H et al.: "Acquisition of HIV-1 Resistance in T Lymphocytes Using an ACA-specific E. coli mRNA interferase", Human Gene Therapy, 22 July 2010 (2010-07-22), pages 1-26, XP002609191, DOI: 10.1089/hum.2010.001 Retrieved from the Internet: URL:http://www.liebertonline.com/doi/abs/1 0.1089/hum.2010.001 [retrieved on 2010-11-11]
- RAGHEB J.A. ET AL.: 'Inhibition of human immunodeficiency virus type 1 by Tat/Rev-regulated expression of cytosine deaminse, interferon alpha2, or diphtheria toxin compared with inhibition by transdominant' REV. HUM. GENE THER. vol. 10, no. 1, 1999, pages 103 - 112, XP000914890
- ISHIKAWA H. ET AL.: 'Retrovirus-mediated gene therapy for hepatocellular carcinoma with reversely oriented therapeutic gene expression regulated by alpha-fetoprotein enhancer/promoter' BIOCHEM. BIOPHYS. RES. COMMUN. vol. 287, no. 4, 2001, pages 1034 - 1040, XP002965091
- Pfeifer A AND Verma M: "Virus Vectors and Their Application" In: KNIPE et al.: "Fields Virology", 1 January 2001 (2001-01-01)

## Description

### Technical Field

The present invention relates to a nucleic acid construct useful in the treatment or prevention of diseases using a polypeptide having endoribonuclease activity.

### Background Art

Gene therapy has been developed as a therapeutic method for treating or preventing diseases (including genetic diseases and cancers) caused by "errors in genetic information" in cells by means of addition of correct genetic information in order to modify the function of the cells or by introducing a novel "protective gene" that is intrinsically absent in the cells.

At present, in addition to the above aspect, utilization of a gene encoding a product exhibiting cytotoxic activity is being extensively studied for the purpose of selective clearance of cells harmful to living organisms (e.g., cancer cells and cells infected with a pathogenic microorganism). For example, expression of a herpes virus thymidine kinase gene in target cells followed by administration of a non-toxic prodrug (gancyclovir) to living organism leads to target cell specific activation of the prodrug, resulting in destruction of the target cells due to the cytotoxicity of the drug.

Toxins encoded by prokaryotic toxin-antitoxin genes are known as polypeptides exhibiting cytotoxic activity. Both MazF, a toxin of the mazE-mazF system (non-Patent Document 1), and PemK, a toxin of the pemI-pemK system (non-Patent Document 2), are known to have single-stranded RNA-specific endoribonuclease activity that recognizes particular nucleotide sequences, and use of these toxins has been proposed to treat proliferative diseases (Patent Document 1).

A human immunodeficiency virus (HIV) infects cells expressing CD4 molecules, such as T cells. This leads to a decrease in CD4-positive T cell (helper T cell) count and a reduction in cellular immunity in humans infected with HIV, finally resulting in severe immunodeficiency, which leads to emergence of opportunistic infections such as Carinii pneumonia. This stage of the disease is referred to as acquired immunodeficiency syndromes (AIDS).

Antiviral agents (such as reverse transcriptase inhibitors, protease inhibitors) and vaccines that interfere with the life cycle of HIV have been developed for treatments of HIV infection, and several antiviral agents have already been put into practical use. However, they cannot necessarily be regarded as a complete set of specific remedies because mutants resistant to these agents may emerge in individuals infected with HIV, which has a high mutation rate. No attempt to develop a gene therapy agent for inhibiting the proliferation of HIV has been accomplished, as another approach, using a nucleic acid, such as an RNA decoy and a ribozyme, or a protein, such as a transdominant mutant protein and an intracellular antibody, as an active ingredient.

Further, methods for causing HIV-infected cell-specific cell death have been contemplated (e.g., Patent Document 2, non-Patent Documents 3, 4 and 5). These methods attempt to connect a gene encoding a cytotoxic product downstream of an LTR promoter derived from HIV. However, up to now, there is no known clinical application of these methods.

Patent Document 3 discloses use of a single-stranded RNA-specific endoribonuclease as a cytotoxic product as mentioned above. This method induces expression of a single-stranded RNA-specific endoribonuclease in a manner depending on Tat protein, a protein expressed in association with HIV infection, and thereby leading to degradation of single-stranded RNA in the cells harboring the HIV genome. This results in inhibition of replication and budding of HIV in the cells. The expression of the endoribonuclease terminates when the expression of Tat protein is terminated by the degradation of HIV-derived RNA and the expressed Tat protein is eliminated from the cells. During this process, ribosomes and tRNA remain intact in the cells and normal protein synthesis is restored once the endoribonuclease expression is terminated. Thus, the cells remaining undestroyed at this time point restores the proliferation. As such, while this method has an advantage in that it does not lead to excessive decrease in CD4-positive T-cells, it may fail to introduce an endoribonuclease gene into cells at a sufficient efficiency when the construction of the vector is inadequate.
Patent Document 1: WO 2004/113498
Patent Document 2: US Patent No. 5,554,528
Patent Document 3: WO 2007/020873
non-Patent Document 1: Molecular Cell, 12: 913-920 (2003)
non-Patent Document 2: J. Biol. Chem., 279: 20678-20684 (2004)
non-Patent Document 3: Hum. Gene Therapy, 2: 53-61 (1991)
non-Patent Document 4: Proc. Natl. Acad. Sci. USA, 91: 365-369 (1994)
non-Patent Document 5: Hum. Gene Therapy, 10: 103-112 (1999)
EP 1 921 136 A1 describes a nucleic acid for the treatment or prevention of immunodeficiency virus infection. Mineno, J., et al. (J. Gene Med., 8: 1465 (2006)) describe the development of anti-HIV gene therapy strategy using the endoribonuclease MazF. Joshi, P.J., et al. (Mol. Ther., 11: 677-686 (2005)) describe that aptamers directed to HIV-1 reverse transcriptase display greater efficacy over small hairpin RNAs targeted to viral RNA in blocking HIV-1 replication.

### Disclosure of the Invention

### Problems to be Solved by the Invention

In view of the foregoing prior art, the present invention has been made. An objective of the present invention is to provide a more effective method for treating and/or preventing diseases using endoribonucleases. Means for Solving the Problems

The present inventors investigated the construction of retroviral vectors for expressing a polypeptide having single-stranded RNA-specific endoribonuclease activity, which can be used for treating and/or preventing a variety of diseases including, for example, cancers and virus infections. As a result, the present inventors have found that retroviral vectors that can achieve particularly highly efficient gene transfer into cells can be obtained if a unit for transcription of the gene encoding the polypeptide is placed in a retroviral vector in the opposite direction relative to the direction of transcription of the RNA genome of the retroviral vector, thereby completing the present invention.

More specifically, the present invention relates to:
[1] A retroviral vector which comprises a transcription unit comprising a transcription regulatory sequence and a gene encoding a polypeptide having single-stranded RNA-specific endoribonuclease activity which is placed so that its expression can be controlled by the regulatory sequence, wherein the unit is placed so that the direction of transcription of mRNA from the unit is opposite to the direction of transcription of RNA genome of the retroviral vector;
[2] The retroviral vector according to [1], wherein the transcription regulatory sequence is a sequence for controlling the transcription of a gene expressed specifically in cancer cells or virus-infected cells;
[3] The retroviral vector according to [1], wherein the transcription regulatory sequence is such a sequence that the transcription is induced by a trans-acting factor of a cancer cell or virus origin;
[4] The retroviral vector according to [3], wherein the transcription regulatory sequence is such a sequence that the transcription is induced by a trans-acting factor of an immunodeficiency virus;
[5] The retroviral vector according to [4], which comprises such a transcription regulatory sequence that the transcription is induced by Tat protein and/or Rev protein;
[6] The retroviral vector according to [4], wherein a gene encoding a polypeptide having single-stranded RNA-specific endoribonuclease activity is placed downstream of the LTR of an immunodeficiency virus;
[7] The retroviral vector according to [1], wherein the transcription regulatory sequence is such a sequence that the transcription is induced by a trans-acting factor artificially supplied into cells;
[8] The retroviral vector according to any one of [1] to [7], wherein the polypeptide having single-stranded RNA-specific endoribonuclease activity is MazF protein.;
[9] The retroviral vector according to any one of [1] to [8] for use in a method for treating or preventing a disease;
[10] The retroviral vector for use in a method for treating or preventing a disease according to [9], wherein the disease is a cancer or a virus infection;
[11] The retroviral vector for use in a method for treating or preventing a disease according to [9], wherein the disease is an immunodeficiency virus infection;
[12] A therapeutic or preventive agent of a disease, which comprises the retroviral vector according to any one of [1] to [8] as an effective component;
[13] The therapeutic or preventive agent according to [12] for use in a method of treating or preventing cancer or virus infection; and
[14] The therapeutic or preventive agent according to [13] for use in a method of treating or preventing an immunodeficiency virus infection.

### Effect of the Invention

According to the present invention, a retroviral vector is provided which is highly effective for gene therapy that utilizes expression of single-stranded RNA-specific endoribonuclease activity in the cells. The retroviral vector is useful for the treatment and/or prevention of cancers and virus infections, particularly RNA-virus infections.

### Brief Description of Drawings

Fig. 1 illustrates the construction of pMT-HLTR-MazF-PL.
Fig. 2 illustrates the construction of pMTD3-U3TAR-MazF-PL.
Fig. 3 illustrates histograms of the numbers of cells infected and uninfected with HIV.

### Best Mode for Carrying Out the Invention

As used herein, the term "single-stranded RNA-specific endoribonuclease activity" refers to activity that hydrolyzes the phosphodiester bond at the 3'-side of a ribonucleotide in a single-stranded nucleic acid containing at least one ribonucleotide molecule as a constituent nucleotide. A nucleic acid hydrolyzed by this activity forms a 3'-end having a hydroxyl group and a 5'-end having a phosphate group, a 3'-end having a phosphate group and a 5'-end having a hydroxyl group, or a 5'-end having a 2',3'-cyclic phosphate and a hydroxyl group. Any polypeptide which is unable to cleave duplex nucleic acids such as double-stranded RNAs or RNA-DNA hybrids can be used in the present invention, though the present invention is not specifically limited thereto. The above-mentioned substrate specificity is suitable for the present invention in view of efficient degradation of the HIV genome which is a single-stranded RNA. Those having activity of cleaving an RNA in a nucleotide sequence-specific manner or those capable of degrading a mRNA in a ribosome-independent manner are particularly preferred for use in the present invention. Examples of polypeptides having single-stranded RNA-specific endoribonuclease activity (hereinafter, sometimes, referred to as a single-stranded RNA-specific endoribonuclease) include enzymes called mRNA interferases such as MazF and PemK described hereinafter (WO 2004/113498).

The polypeptides used in the present invention which have activity of degrading single-stranded RNA in sequence-specific and ribosome-independent manners can be, for example, those of a microorganism origin, though the present invention is not specifically limited thereto. In such a case, a gene encoding the above-mentioned polypeptide can be isolated from a microbial genome or a plasmid. For example, in the present invention, there can be used genes encoding endoribonucleases, MazF and PemK, which are constituent toxins of a toxin-antitoxin system and are described in Molecular Cell, 12:913-920 (2003) and J. Biol. Chem., 279:20678-20684 (2004), respectively. MazF is an enzyme that cleaves the sequence 5'-A/CA-3' in a single-stranded RNA, and PemK is an enzyme that primarily cleaves the sequence 5'-U/A(C, A or U)-3'. It is also possible to select a gene encoding an amino acid sequence highly homologous to the amino acid sequence of MazF or PemK from a known database to isolate this and use it in the present invention. Polypeptides having the above-mentioned activity have been found in a large number of microorganisms including blue-green algae and archae (WO 2006/123537, WO 2007/010740, WO 2007/013264, WO 2007/013265, etc). Preferably, a gene encoding MazF is used in the present invention. The amino acid sequence of MazF is shown in the Sequence Listing as SEQ ID NO. 1.

The endoribonucleases described above, constituent toxins of a toxin-antitoxin system, are suitable for use in the present invention because they are not inhibited by any cytoplasmic ribonuclease inhibitor such as a human placental ribonuclease inhibitor (WO 2004/113498). If the endoribonucleases are persistently expressed, new protein synthesis does not take place in cells, resulting in inhibition of cell growth followed by induced cell death. Further, synthesis of viral component proteins is blocked in virus-infected cells, and replication or budding of the virus does not occur. Thus, the retroviral vector of the present invention allows for clearance of cancer cells or virus-infected cells from living organisms. In particular, in cells infected with an RNA virus, the viral genome itself is degraded by the above-mentioned endoribonuclease.

The retroviral vector of the present invention is that containing a transcription unit comprising a transcription regulatory sequence and a gene encoding a polypeptide having single-stranded RNA-specific endoribonuclease activity which is placed so that its expression can be controlled by the regulatory sequence, and is characterized in that the unit is placed so that the direction of the transcription of a mRNA from the unit is opposite to the direction of transcription of the RNA genome of the retroviral vector.

As used herein, the term "transcription regulatory sequence" refers to a nucleic acid sequence that is capable of controlling the transcription of a gene linked downstream therefrom. Examples of the transcription regulatory sequence include a promoter that controls the transcription in an on/off manner mediated by association/dissociation of a particular trans-acting factor, though the present invention is not specifically limited thereto. The nucleic acid located between the loxP sequences derived from P1-phase can be used as a Cre recombinase-inducible transcription regulatory sequence by inserting the nucleic acid into a site between any promoter and a gene of interest.

In the present invention, there can be used any sequence (promoter) that controls transcription of a gene expressed specifically in cancer cells or cells infected with a pathogenic microorganism, and any sequence that controls transcription induced by a trans-acting factor present specifically in cancer cells or virus-infected cells. Further, by combining the artificial supply of a trans-acting factor into the cells, any transcription regulatory sequence with which transcription is induced by the trans-acting factor can be used. "The transcription regulatory sequence with which transcription is induced by the trans-acting factor" as described above may be a sequence that has a region intrinsically capable of interacting with the factor, or may be an artificial combination of a sequence having a region capable of interacting with the factor and another independent sequence having transcriptional activity.

Examples of the transcription regulatory sequences for the genes specifically expressed in cancer cells include tumor-specific promoters (promoters originated from the genes of AFP, CEA, PSA, midkine, telomerase, MAGE, tyrosinase, IAI.3B, etc). Further, examples of the trans-acting factors present specifically in virus-infected cells include human immunodeficiency virus-originated Tat protein and Rev protein. The Tat and Rev proteins induce the transcription of genes under the control of TAR (trans-activation responsive elements) sequences and RRE (Rev-responsible elements) sequences, respectively.

In the aspect in which a trans-acting factor is artificially supplied into cells, it is preferred to use a transcription regulatory sequence that normally shows no transcription-inducing activity in the cells. Since such a sequence allows transcription of a gene located downstream thereof only when the trans-acting factor is supplied, selective expression of a gene of interest (i.e., a gene encoding a polypeptide having single-stranded RNA-specific endoribonuclease activity) is achieved in target cells. Supply of trans-acting factors into cells can be achieved using a method for introducing the factors into cells directly or by means of a suitable auxiliary agent, or by introducing the factors into cells using any well-known method for introducing a gene encoding the factor into the cells.

As described above, the retroviral vector of the present invention is useful for clearance of cancer cells and/or virus-infected cells from living organisms. When a single-stranded RNA-specific endoribonuclease is expressed in cells infected with an RNA virus, the genomic RNA of the virus generated by viral replication is cleaved by the polypeptide described above, thereby inhibiting the viral replication. Therefore, the retroviral vector of the present invention is particular useful for treating and/or preventing RNA virus infections such as infections caused by retroviruses (e.g., human T-lymphotropic virus), lentiviruses (e.g., immunodeficiency virus), flaviviruses (e.g., hapatitis C virus and Japanese encephalitis virus), bunyaviruses (e.g., hanta virus), influenza viruses, and coronaviruses (e.g., SARS).

As one aspect of the present invention, a retroviral vector is exemplified which contains a transcription unit comprising a transcription regulatory sequence which is such a sequence that the transcription is induced by an immunodeficiency virus-originated transacting factor, and a gene encoding a polypeptide having single-stranded RNA-specific endoribonuclease activity which is placed so that that expression can be controlled by the transcription regulatory sequence. Such retroviral vector is useful for treating and/or preventing an immunodeficiency virus infection.

Hereinafter, this aspect will be described in detailed.
As used herein, the term "immunodeficiency virus" refers to a virus that causes acquired immunodeficiency by infecting and destroying immune cells. Examples of immunodeficiency viruses include human immunodeficiency virus (HIV), simian immunodeficiency virus (SIV), and feline immunodeficiency virus (FIV). In addition, chimera viruses formed by combining different immunodeficiency virus genomes are also included in the "immunodeficiency viruses" described herein. Examples of such chimera viruses include simian/human immunodeficiency virus (SHIV).

Further, as used herein, the immunodeficiency virus infection refers to a pathological condition in which acquired immunodeficiency is caused by the above immunodeficiency virus.

Although the transcription regulatory sequence with which transcription is induced by an immunodeficiency virus-originated trans-acting factor is not specifically limited, a transcription regulatory sequence with which transcription is induced by immunodeficiency virus-originated Tat protein can be used. Such sequence is preferably a transcription sequence with which transcription is induced by Tat protein originated from an immunodeficiency virus, for example, HIV. Since Tat protein binds to a TAR (trans-activation responsive element) sequence present in a RNA whose transcription is initiated by the action of the LTR promoter of an immunodeficiency virus to activate the transcription of the downstream sequence thereof, in the present invention, there can be used a transcription regulatory sequence that has the nucleotide sequence of the TAR region downstream of the transcription initiation site. Particularly preferred transcription regulatory sequence to be used is the LTR of the immunodeficiency virus (e.g., HIV) to be treated or prevented or a suitably modified LTR. Examples of the modifications include a deletion of a host cell-originated transcription factor biding site in the promoter and a deletion of a region unnecessary for Tat protein-specific transcription. The former modification permits a reduction in the level of the transcription by a transcription factor originated from the host cell, which is irrelevant to infection with an immunodeficiency virus such as HIV. Such modification in a transcription regulatory sequence is described, for example, in non-Patent Document 2. Further, examples of the latter modification include a deletion of the U5 region or a region downstream of the TAR sequence in LTR (a part of the R region and the U5 region).

Further, RRE (Rev-responsible element) present in the immunodeficiency virus genome is known to enhance protein expression by interacting with the Rev protein, an immunodeficiency virus-originated trans-acting factor (non-Patent Document 3). It is further known that, although the region called INS present within the gag and pol genes inhibits transcription of an immunodeficiency virus mRNA in the absence of the Rev protein, this inhibition is removed by the interaction between the RRE and the Rev protein (J. Virol., 66: 7176-7182 (1992)). Thus, incorporation of these transcription regulatory sequences into the 3'-terminal region of the gene encoding a foreign polypeptide permits the expression of the polypeptide encoded by the nucleic acid in a Rev protein-dependent or immunodeficiency virus infection-dependent manner.

The above-described sequence that interacts with the immunodeficiency virus-originated trans-acting factor can be used in combination with a functional sequence into which the transacting sequence is inherently incorporated (e.g., a promoter) or in combination with a heterologous functional sequence. For example, a sequence constructed by combining a promoter that is capable of initiating mRNA transcription in desired cells and not originated from immunodeficiency virus with the above-described sequence is included in the "transcription regulatory sequences" used in the present invention.

The endoribonuclease described above does not degrade a rRNA as a constituent of a ribosome or a tRNA folded into a higher order structure. Therefore, if a gene encoding the above-described enzyme is placed under the control of a transcription regulatory sequence with which transcription is induced by an immunodeficiency virus-originated trans-acting factor, an RNA of a RNA virus is degraded and eliminated in cells into which the retroviral vector of the present invention is introduced, resulting in termination of the endonuclease expression in the cells, which permits the cells to restore the proliferative potential. Cells in which viral replication is inhibited retain their proliferative potential, even if an immunodeficiency virus, for example, is integrated into the chromosomes of the cells as a provirus. The use of the endoribonuclease is advantageous in that replication of an immunodeficiency virus and onset of immunodeficiency diseases can be prevented without decreasing the number of T cells in the living organism.

An endoribonuclease that is a constituent toxin of a toxin-antitoxin system cleaves a single-stranded RNA by recognizing a short nucleotide sequence about 3 to 7 nucleotides in length. Thus, if the endoribonuclease is expressed in cells, most of the cellular mRNAs are degraded, thereby inhibiting protein synthesis in the cells and the cell growth. The endoribonuclease also cleaves the immunodeficiency virus genomic RNA, and the replication and extracellular release (budding) of the above-described virus (e.g., HIV) are also prevented. Furthermore, expression of the polypeptide encoded by the immunodeficiency virus genome is also inhibited, which prevents, for example, extracellularly released Tat protein from inducing apoptosis of cells uninfected with the immunodeficiency virus.

For the treatment or prevention of immunodeficiency virus infection, it is desirable to introduce a gene as used in the present invention which encodes a polypeptide having single-stranded RNA-specific endoribonuclease activity into cells potentially infected with immunodeficiency virus, for example, cells (or a cell population) including CD4-positive cells in humans and primates. Thus, in the present invention, gene transfer is carried out targeting CD4-positive cells (e.g., T-cells), precursor cells capable of differentiating into CD4-positive cells (e.g., hematopoietic stem cells), or a cell population containing those cells. It is preferred to target hematopoietic stem cells or a cell population containing these cells in the present invention, from the viewpoint of conducting comprehensive transfer of the gene into the cells that are potentially infected with an immunodeficiency virus. The above-described cells are not specifically limited as long as they contain CD4-positve cells or precursor cells thereof. Examples thereof include blood cells (peripheral blood cells, umbilical cord blood cells) and bone marrow cells collected from an individual or CD4-positive cells, precursor cells of CD4-positive cells and hematopoietic stem cells fractionated from the above-described cells by a known method.

As described hereinabove, the present invention is useful for producing a cell useful for the treatment or prevention of immunodeficiency virus infection, and for producing a cell composition comprising the cell. Furthermore, according to the present invention, the cell can be used in a method for treating or preventing immunodeficiency virus infection. Specifically, administration of cells introduced with the retroviral vector of the present invention to an individual can reduce the rate of immunodeficiency virus-infected cells in the individual or confer resistance to immunodeficiency virus infection on the individual. The above-described cell and cell composition comprising the cell are useful for treating or preventing particularly HIV infection or AIDS.

In the CD4-positive cells differentiated from CD4-positive cells introduced with the retroviral vector of the present invention or from precursor cells (e.g., a hematopoietic stem cell) of CD4-positive cells introduced with the retroviral vector of the present invention, expression of a gene encoding a polypeptide having single-stranded RNA-specific endoribonuclease activity that is placed downstream of a transcription regulatory sequence is induced upon infection with an immunodeficiency virus and subsequent formation of a trans-acting factor originated from the immunodeficiency virus in the cells. As a result, the polypeptide produced degrades mRNAs in the cells, thereby inhibiting protein biosynthesis. During this process, the RNA genome being formed through the replication process is also subjected to the degradation. Thus, since both translation of the polypeptide encoded by the immunodeficiency virus genome and replication of the immunodeficiency virus genome are inhibited, both production of new infectious immunodeficiency virus particles and infection of other cells will be prevented. Furthermore, if cells introduced with the retroviral vector of the present invention are cultured by mixing with immunodeficiency virus-infected cells in which the immunodeficiency virus is produced extracellularly, the number of the cells introduced with the retroviral vector of the present invention will increase while the number of the cells into which the vector is not introduced will decrease. As a result, the rate of immunodeficiency virus-producing cells will be reduced.

Furthermore, according to the present invention, the pharmaceuticals described above may be used in a method for treating and/or preventing immunodeficiency virus infection. The therapeutic method can be carried out either alone or in combination with multidrug therapy (HAART), which include combined use of three to four different antiviral drugs (e.g., reverse transcription inhibitors and protease inhibitors).

The retroviral vector of the present invention comprises a nucleic acid in which a gene encoding a polypeptide having single-stranded RNA-specific endoribonuclease activity which is placed so that expression can be controlled by the regulatory sequence is linked to the transcription regulatory sequence described above, i.e., a single-stranded RNA-specific endoribonuclease transcription unit. In the present invention, the transcription unit is placed so that the direction of the transcription of an mRNA from the unit is reverse to the direction of the transcription of the RNA genome of the retroviral vector.

A retrovirus is a single-stranded RNA virus in which genome 5'-LTR, a packaging signal, the gag, pol, and env genes, and 3'-LTR are found as major elements in the 5' to 3' order. Among these elements, the 5'-LTR, packaging signal and 3'-LTR are essential for a retroviral vector. In a retroviral vector used for introduction of an exogeneous gene into a cell, usually, genes such as gag, pol, and env are deleted and instead, a desired gene is inserted. In general, a gene of interest is inserted into a retroviral vector in the same direction as that of the transcription of the viral genome, and the gene of interest is transcribed from the 5'-LTR promoter or an internal promoter inserted upstream of the gene of interest. However, when a single-stranded RNA-specific endoribonuclease gene is inserted in the same direction as that of the transcription of the retroviral genome, the endoribonuclease gene is expressed from the retroviral mRNA, and high-titer production of retroviral vectors cannot be expected because the endoribonuclease destroys the viral genome RNA (mRNA).

In the present invention, the single-stranded RNA-specific endoribonuclease transcription unit is placed in the direction opposite to the direction of the transcription of the RNA genome in the retroviral vector. That is, the direction of transcription initiated by the transcription regulatory sequence of the transcription unit is opposite to the direction of transcription initiated from the 5'-LTR of the retroviral vector. Therefore, this means that the retroviral mRNA is equivalent to the antisense chain of the endoribonuclease gene, and the endoribonuclease will never be expressed from this mRNA. Further, even if weak transcription of the endoribonuclease mRNA may occur due to the property of the transcription regulatory sequence, expression of the endoribonuclease will be inhibited because the retroviral mRNA functions as antisense RNA.

The retroviral vector to be used in the present invention is not specifically limited. A replication-defective retroviral vector is preferred for the present invention from the viewpoint of preventing unlimited infection or gene transfer. The vector lacks the ability to replicate autonomously in infected cells and therefore is nonpathogenic. The vector can enter host cells such as vertebrate cells, in particular, mammalian cells, to stably integrate a foreign gene, which is inserted into the vector, into the chromosomal DNA of the cells. Example of the known replication-defective retroviral vector include retroviral vectors such as MFG and α-SGC vectors (WO 92/07943), pBabe (Nucleic Acids Research, 18: 3587-3596 (1990)), pLXIN (Takara Bio Inc.) or pDON-AI (Takara Bio Inc.), lentivirus vectors and modifications thereof. Examples of the lentivirus vector include, but is not specifically limited to, human immunodeficiency virus (HIV)-derived vector (HIV vector having a wild-type LTR (e.g., US Patent No. 5,665,577) and HIV vector having a modified LTR (e.g., pLenti6/V5, Invitrogen Corporation), and simian immunodeficiency virus (SIV)-originated vector (e.g., Human Gene Therapy, 11: 1863-1874 (2000)).

The retroviral vector of the present invention can be constructed by selecting a suitable vector from the retroviral vectors described above and loading a single-stranded RNA-specific endoribonuclease transcription unit onto the vector. The preparation method of the retroviral vector constructed is not specifically limited, and it can be obtained by culturing retrovirus-producer cells introduced with the retroviral vector constructed and collecting the culture supernatant. The retrovirus producer cells may be cells that produce retrovirus particles into the supernatant in a stable manner, or may be those transiently producing the retrovirus particles by transfection with a retroviral vector plasmid.

For the preparation of retrovirus producer cells, any known packaging cell line, such as PG13 (ATCC CRL-10686), PA317 (ATCC CRL-9078), GP+E-86 or GP+envAm-12 (US Patent No. 5,278,056), or Psi-Crip (Proc. Natl. Acad. Sci. USA, 85: 6460-6464 (1988)) can be used. Also, retrovirus-producer cells can be prepared using 293 cells or 293T cells, which have high transfection efficiency.

The present invention can use a retrovirus prepared by means of pseudotyped packaging. Such a retrovirus has the envelope of different origin from the virus from which its genome originated. Examples of the pseudotyped retrovirus which can be used include that having an envelope originated from Moloney murine leukemia virus (MoMLV), gibbon ape leukemia virus (GaLV), vesicular stomatitis virus (VSV) or feline endogenous virus, or a protein that can function as the envelope. Furthermore, a retroviral vector having a glycosylated protein on its surface can be used. Such a vector is prepared using a retrovirus-producing cells into which a gene encoding an enzyme involved in glycosylation.

In addition to the single-stranded RNA-specific endoribonuclease transcription unit, the retroviral vector of the present invention can contain elements acting in concert with the transcription regulatory sequence of the unit, such as operator, enhancer and terminator sequences. Further, independently from the unit, the retroviral vector of the present invention can have an appropriate marker gene that allows selection of transgenic cells (e.g., a drug resistance gene, a gene encoding a protein that can function as a reporter), a receptor gene, and the like. Furthermore, the retroviral vector of the present invention can be loaded with a suicide gene for the purpose of eliminating the transgenic cells *in vivo* once the treatment with the transgenic cells has been completed, or in the case where there occurs any side effect. These genes are not necessarily required to be placed so that they are transcribed in the direction reverse to the direction of transcription of the RNA genome.

The activity of an endoribonuclease that is a constituent toxin of a toxin-antitoxin system such as MazF or PemK is inhibited in the coexistence of the corresponding antitoxin (e.g., MazE or PemI). Thus, when a toxic endoribonuclease gene is used in the present invention, expression of the cytotoxicity caused by the endoribonuclease activity in HIV-uninfected cells can be prevented by using an antitoxin gene in combination. As an example of the aspect described above, to inhibit the toxin activity by using the antitoxin activity, it is contemplated that a trace amount of the antitoxin is left to be expressed in HIV-uninfected cells by linking the antitoxin gene downstream of, for example, a constitutive promoter (e.g., a promoter derived from a housekeeping gene such as a glyceraldehyde 3-phosphate dehydrogenase or β-actin gene, or a virus-derived promoter). Once such cells are infected with HIV, expression of the toxin is stimulated by the action of a trans-acting factor as described above, and the cytotoxicity is exhibited only when the amount of expressed toxin exceeds that of the expressed antitoxin. Thus, the selectivity of cytotoxicity to HIV-infected cells can be improved.

In one aspect of the present invention, *ex vivo* gene transfer is used by which the retroviral vector of the present invention is introduced *ex vivo* into the cells collected from a biological individual. When a viral vector is used, the gene transfer is achieved by mixing the above target cells, collected from a biological sample, with the retroviral vector of the present invention, for example, with a culture supernatant of the virus-producing cells or with the retroviral vector purified from such culture supernatant, and then incubating the resultant mixture under appropriate conditions. Thus, the cells having a single-stranded RNA-specific endoribonuclease transcription unit integrated into their chromosomes can be prepared. Another aspect of the present invention is *in vivo* gene transfer method by which the retroviral vector of the present invention is administered directly to individuals.

If the retroviral vector is used for *ex vivo* gene transfer, the retroviral vector can infect the target cells with high efficiency in the presence of a functional substance having retrovirus-binding activity.

The gene transfer method using a functional substance having retrovirus-binding activity is disclosed, for example, in WO 95/26200, WO 97/18318, and Nature Medicine, 2: 876-882 (1996). Such methods are divided into a method using a functional substance having both a retrovirus-binding site and a target cell-binding site in a single molecule, and a method using a mixture of a functional substance having a retrovirus-binding site and a functional substance having a target cell-binding site. Both methods can be used in the present invention.

The above-described functional substance is not specifically limited as long as it has retrovirus-binding activity and/or target cell-binding activity. Examples of the functional substances having retrovirus-binding activity include the fibronectin heparin-binding domain (heparin-II domain), fibroblast growth factor, type V collagen fragment, a derivative or a variant of the polypeptides, polylysine, and DEAE-dextran. Further, any substance capable of binding to desired target cells can be used as a functional substance having target cell-binding activity. Examples of the functional substances having target cell-binding activity include polypeptides having cell-binding activity (e.g., cytoskeletal proteins), antibodies that recognize a cell or a biological molecule on the cell surface, growth factors, cytokines, and sugar chains, although the present invention is not specifically limited thereto.

As one preferable aspect of the present invention, there is a method wherein gene transfer into target cells is carried out in the presence of a functional substance including the fibronectin-derived heparin-binding domain. By way of example, the functional substance is preferably a fibronectin fragment having both the cell adhesion domain and the heparin-binding domain. As the cell adhesion domain, VLA-5 and/or VLA-4-binding region polypeptides are particularly preferred. A fibronectin fragment as described above can be prepared from fibronectin purified from a living organism by certain means, such as protease digestion, or by using recombinant DNA technology. For example, the recombinant fibronectin fragment commercially available from Takara Bio Inc. in the name of RetroNectin (registered trademark) is preferable for the present invention.

As described hereinabove, the retroviral vector of the present invention, and the cells introduced with the retroviral vector can be used for treating and/or preventing cancer and virus infection. That is, the present invention provides an agent for treating and/or preventing the above-described diseases, for example, RNA-virus infections (e.g., immunodeficiency virus infection (AIDS) and hepatitis C). Such an agent is not specifically limited as long as it comprises the retroviral vector or cells as an active ingredient. Such an agent can also take any forms, and in addition to as the active ingredient alone, for example, it can be in the form of a preparation comprising the active ingredient in combination with a pharmaceutically acceptable carrier, and a kit for *ex vivo* gene transfer into a cell using the vector.

As described in Examples hereinafter, in the populations of cells into which an endoribonuclease-encoding gene is introduced by the retroviral vector of the present invention, HIV replication is reduced to about 1/5 as compared with that in a cell population free of the cells harboring the gene, even if only about 50% of the cells in the infected cell population retain the gene. Thus, the therapeutic and/or preventive effect produced by the present invention is not limited by the introduction efficiency of a gene that functions as the active agent. Although the present invention is not limited to any particular theory, the gene therapy by using the retroviral vector of the present invention for the treatment of HIV has such an unexpectedly advantageous feature as a chain reaction mechanism, wherein a therapeutic gene or the protein expressed therefrom is transmitted from transformed to nontransformed cells without being mediated by a vector.

### Examples

Hereinafter, the present invention will be illustrated in more detail with reference to Examples, but is not to be construed to be limited thereto.

### Example 1

### Construction of MazF expression plasmids

### (1) Construction of MazF-expressing retroviral vector plasmid pMT-HLTR-MazF-PL

The region encoding gag, sgGFP and RRE inserted into plasmid pQBI-LTRgagGFP (Quantum Biotechnologies Inc.) was removed by double digestion with the restriction enzymes SalI and XbaI, and a chemically synthesized DNA having the nucleotide sequence shown by SEQ ID NO: 2 in the Sequence Listing, and encoding the MazF protein was inserted into the plasmid between the SalI and XbaI sites. The plasmid thus constructed was designated pQBI-LTRMazFcvl. A region extending from the HIV-LTR to the MazF-coding region was amplified by PCR using pQBI-LTRMazFcv1 as the template and the two primers shown by SEQ ID NOs: 3 and 4. The amplified fragment thus obtained was digested with the restriction enzymes NotI and Eco52I. This DNA fragment was designated and named HIV-LTR-MazF cassette.

A DNA fragment containing the herpes simplex virus type 1 poly(A) signal site was amplified by PCR using the plasmid pZsGreen1-N1 (Takara Bio Inc.) as the template and the two primers shown by SEQ ID NOs: 5 and 6. The amplified fragment thus obtained was digested with the restriction enzymes MluI and NotI. This DNA fragment was named pA fragment.

A fragment containing a mouse PGK promoter was amplified by PCR using pQBI-pgk(Quantum Biotechnologies Inc.) as the template and the primers shown by SEQ ID NOs: 7 and 8. The amplified fragment thus obtained was digested with the restriction enzyme MluI, followed by blunting and further digestion with BamHI. This DNA fragment was named pPGK fragment.

The extracellular domain of human Low affinity Nerve Growth Factor Receptor was amplified by PCR using the genome of the retrovirus-producing cell SFCMM-3 (Human Gene Therapy, 9: 2243-2251 (1998)) as the template and the primers shown by SEQ ID NOs: 9 and 10. The amplified fragment thus obtained was digested with the restriction enzymes EcoRI and XhoI. This DNA fragment was named LNGFR fragment.

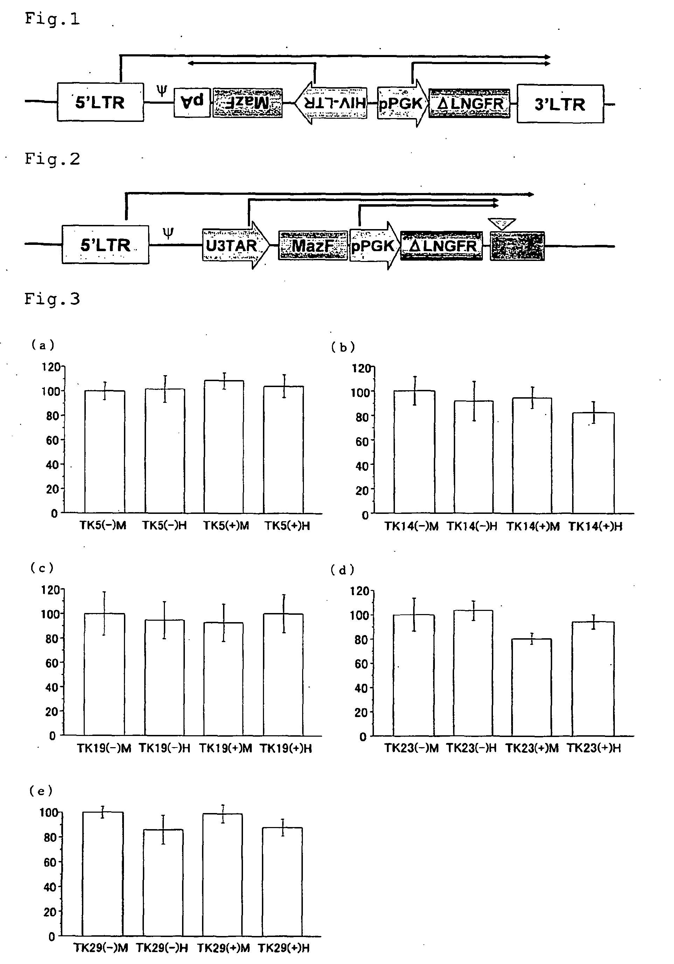

The gene fragments described above were inserted in turn into pMT vector (Gene Therapy, 11: 94-99 (2004)) between the MluI and BamHI sites to obtain the recombinant retroviral vector plasmid pMT-HLTR-MazF-PL, shown in Fig. 1. This vector is characterized in that the HIV-LTR-MazF cassette is inserted in the direction opposite to the direction of the transcription of the retroviral vector genome.

### (2) Construction of MazF-expressing retroviral vector plasmid pMTD3-U3TAR-MazF-PL

For the purpose of comparing with pMT-HLTR-MazF-PL, a self-inactivating vector, pMTD3-U3TAR-MazF-PL, was constructed, wherein the HIV-LTR-MazF cassette was inserted in the same direction as that of the transcription of the retroviral vector genome so that, after the gene transfer, the transcription of the retroviral genome was inhibited (Fig. 2). For preparing this vector, the plasmid vector pMTD3 in which the portion extending from the nucleotide 43 to the nucleotide 309 of the 3'LTR U3 region of vector pMT was deleted was used. The DNA fragment prepared as described above in (1) was inserted between the MluI and BamHI sites of this vector as shown in Fig. 2.

Further, a vector expressing MazE, i.e., an antitoxin of MazF, was constructed as follows. Initially, the CMV promoter of vector pIRESneo3 (Takara Bio Inc.) was replaced with the CAG promoter to prepare vector pCAIN. Subsequently, the gene encoding the MazE protein having the amino acid sequence shown by SEQ ID NO: 11 (having the nucleotide sequence shown by SEQ ID NO: 12) was inserted between the EcoRI and NotI sites of pCAIN. The vector thus constructed named pCAIN-MazE.

### Example 2

Establishment of retrovirus producer cells and preparation of retroviral vector
Transient virus production was carried out using pMT-HLTR-MazF-PL and Retrovirus Packaging Kit Eco (Takara Bio Inc.) to obtain the ecotropic virus MT-HLTR-MazF-PL. This ecotropic was allowed to infect GaLV retrovirus packaging PG13 cells (ATCC CRL-10686) in the presence of 8 µg/mL polybrene (Sigma-Aldrich Japan K.K.) to obtain cells for gene transfer. The cells were plated in a 96 well-plate by limiting dilution, and the culture supernatants of the proliferated clones were removed to determine the virus titers using real-time PCR as described in Example 3. Clone 1, in which production of high-titer virus was observed, was selected to establish the retrovirus producer cell line PG13/MT-HLTR-MazF-PL.

PG13/MT-HLTR-MazF-PL was cultured in Dulbecco's modified Eagle's medium (DMEM; Sigma-Aldrich Japan K.K.) supplemented with 10% fetal bovine serum (Invitrogen Corporation). When the culture reached semiconfluence, the medium was replaced with fresh DMEM medium (0.1mL/cm²) containing 10% fetal bovine serum supplemented with 5 mM sodium butyrate. After culturing for 24 hours, the supernatant was filtrated through 0.45 µm filter (Millipore Corporation) to obtain a solution of recombinant retrovirus GaLV/MT-HLTR-MazF-PL. This virus solution was dispensed in small aliquots and stored in the freezer at -80°C. These aliquots were used in the gene transfer study described below.

pMTD3-U3TAR-MazF-PL and pCAIN-MazE were mixed at a ratio of 20:1. PG13 cells were transfected with this mixture using LipofectAMINE 2000 (Invitrogen Corporation) and selected in DMED medium containing 10% fetal bovine serum supplemented with 500 µg/mL neomycin (G418). The G418-resistant cell population thus obtained was subjected to a separation procedure using CD271 LNGFR MicroBead Kit (Miltenyi Biotec K.K.) to obtain LNGFR-positive cells. The LNGFR-positive cells were plated in a 96 well-plate by limiting dilution, and the culture supernatants of the proliferated clones were removed to determine the virus titers using real-time PCR as described in Example 3. Thus, the cell line PG13/MTD3-U3TAR-MazF-PL producing a high-titer viral vector was established. PG13/MTD3-U3TAR-MazF-PL was cultured in DMEM medium supplemented with 10% fetal bovine serum. When the culture reached semiconfluence, the medium was replaced with fresh DMEM medium (0.1mL/cm²) containing 10% fetal bovine serum supplemented with 5 mM sodium butyrate. After culturing for 24 hours, the supernatant was filtrated through 0.45 µm filter (Millipore Corporation) to obtain a solution of recombinant retrovirus GaLV/MTD3-U3TAR-MazF-PL. This virus solution was dispensed in small aliquots and stored in the freezer at -80°C.
These aliquots were used in the gene transfer study described below.

### Example 3

### Determination of virus titer

Real-time PCR reaction was carried out using 1 µl of a virus solution as a template and using Retro-X qRT-PCR Titration Kit (Takara Bio Inc.), thereby calculating the number of RNA copies in the virus solution from a calibration curve. The titers of the virus solutions obtained in Example 2 were 9.78 x 10⁹ copies/mL for GaLV/MT-HLTR-MazF-PL and 1.16 x 10⁹ copies/mL for GaLV/MTD3-U3TAR-MazF-PL. By loading the HLTR-MazF expression cassette in the reverse direction, it became possible to obtain a recombinant virus with higher titer than a self-inactivating retroviral vector.

### Example 4

### Preparation of CH-296 coated plates

To each well of a 24-well plate without surface treatment (Falcon), 500 µl of a fibronectin fragment, CH-296 (product name: RetroNectin; Takara Bio Inc.), was added at a concentration of 20 µg/ml. The plate was allowed to stand overnight at 4°C, and then washed with PBS. This plate was used as a CH-296-coated plate in the experiments described below.

### Example 5

### Determination of gene transfer efficiency

Gene transfer efficiency was determined by carrying out gene transfer experiment, wherein CD4-positive T cells prepared from the human acute lymphoblastic leukemia cell line CCRF-CEM (ATCC CCL-119) and peripheral blood of cynomolgus monkey were transfected with the recombinant retrovirus solution prepared in Example 2 according to the procedure described below.

CCRF-CEM cells were cultured in RPMI-1640 medium supplemented with 10% fetal bovine serum. On the day of the transfection, the cells were collected by centrifugation and suspended in fresh RPMI-1640 medium supplemented with 10% fetal bovine serum at a density of 1 x 10⁵ cells/mL. To each well of the CH-296-coated plate prepared in Example 4, 1 mL of 4-, 8- and 16-fold dilutions of the recombinant retrovirus solution prepared in Example 2 diluted with RPMI-1640 medium supplemented with 10% fetal bovine serum (Miltenyi Biotec K.K.) were added, and the plate was then centrifuged at 32°C and 2000 x g for 2 hours to facilitate adhesion of the retroviral vector to RetroNectin. The supernatant was then removed and the wells were washed with 0.5 mL of PBS containing 1.5% human serum albumin. Then, 1 mL of the cell suspension described above was added to each of the wells and incubated for three days at 37°C in 5% carbon dioxide atmosphere. After culturing for three days, the cells were collected, stained for LNGFR-positive cells using anti-CD271(LNGFR)-PE antibody (Miltenyi Biotec K.K.), and analyzed by the flow cytometer FACS Vantage (Becton Dickinson and Company) to determine the gene transfer efficiency.

Further, CD4-positive T cells were prepared from the peripheral blood of cynomolgus monkey using Monkey CD4+ T Cell Isolation Kit (Miltenyi Biotec K.K.), and cultured in AIM-V medium (Invitrogen Corporation) supplemented with 10% fetal bovine serum, 2 mM glutamate, 200 U/mL rhIL-2, and 5 µg/ml concanavalin A. After three days, the cells were collected by centrifugation and suspended in fresh AIM-V medium (Invitrogen Corporation) supplemented with 10% fetal bovine serum, 2 mM glutamate and 200 U/mL rhIL-2 at a density of 2 x 10⁵ cells/mL. Two CH-296-coated plates prepared in Example 4 were used. One mL per well of the recombinant retrovirus solution prepared in Example 2 was added to the plates, which were then centrifuged at 32°C and 2000 x g for 2 hours to facilitate adhesion of the retroviral vector to RetroNectin. The supernatant was then removed and the wells were washed with 0.5 mL of PBS containing 1.5% human serum albumin. Then, one of the plates was stored at 4°C without removing the PBS solution containing 1.5% human serum albumin. To the other plate, 1 mL of the cell suspension described above was added to each of the wells and incubated at 37°C in 5% carbon dioxide atmosphere. After 24 hours, the cells were collected, and the cell suspension was added to the virus-adhered plate stored at 4°C. After culturing for another three days, the cells were collected, and stained for LNGFR-positive cells using anti-CD271(LNGFR)-PE antibody (Miltenyi Biotec K.K.), and analyzed by the flow cytometer FACS Vantage (Becton Dickinson and Company) to determine the gene transfer efficiency.

The gene transfer efficiencies thus determined are shown in Table 1. As shown in the table, by loading the HLTR-MazF expression cassette in the opposite direction, the gene transfer efficiency was dramatically improved as compared with the self-inactivating retroviral vector.

**Table 1**

| Retroviral vector | Gene transfer efficiency to CCRF-CEM (%) | | | Gene transfer efficiency to cynomolgus monkey CD4-positive T cells |
|---|---|---|---|---|
| | 4-fold dilution | 8-fold dilution | 16-fold dilution | undiluted solution |
| MT-HLTR-MazF-PL | 77.6 | 81.2 | 79.2 | 85.0 |
| MTD3-U3TAR-MazF-PL | 6.3 | 5.8 | 4.8 | 2.0 |

### Example 6

### Gene transfer to human CD34-positive progenitor cells

Gene transfer efficiency was determined by carrying out gene transfer experiment, wherein human bone marrow CD34-positive progenitor cells (Bone Marrow CD34+ Cells; Cambrex Corporation) were transfected with the recombinant retrovirus solution prepared in Example 2 according to the procedure described below.

Stimulation culture of human CD34-positive progenitor cells was carried out in X-VIVO10 medium (Cambrex Corporation) containing 4% fetal bovine serum supplemented with 20 ng/mL Recombinant human IL-3 (PEPROTECH INC.), 100 ng/mL Recombinant human Stem Cell Factor (PEPROTECH INC.), 100 ng/mL Recombinant human Thrombopoietin (PEPROTECH INC.), and 100 ng/mL Recombinant human Flt3-Ligand(PEPROTECH INC.). After two-days culture at 37°C in 5% carbon dioxide atmosphere, the cells were counted and then suspended in a fresh medium as described above at a density of 1 x 10⁵ cells/mL. To each well of the CH-296-coated plate prepared in Example 4, 1 mL of the undiluted solution of the recombinant retrovirus GaLV/MT-HLTR-MazF-PL prepared in Example 2 was added and the plate was then centrifuged at 32°C and 2000 x g for 2 hours to facilitate adhesion of the retroviral vector to RetroNectin. The supernatant was then removed and the wells were washed with 0.5 mL of PBS containing 1.5% human serum albumin. Subsequently, 1 mL of the cell suspension described above was added to each of the wells and incubated at 37°C in 5% carbon dioxide atmosphere. After culturing for three days, the cells were collected, stained for LNGFR-positive cells using anti-CD271(LNGFR)-PE antibody (Miltenyi Biotec K.K.), and analyzed by the flow cytometer FACS Vantage to determine the gene transfer efficiency. The gene transfer efficiency to human CD34-positive progenitor cells was 85.7%, and the gene transfer with high efficiency was achieved.

### Example 7

### Preparation of CH-296 coated plates

A 24-well plate coated with CH-296 was prepared using the same procedure as that described in Example 4. Further, 1 mL per well of 20 µg/ml CH-295 was added to a 12-well plate without surface treatment (Falcon), and the plate was allowed to stand overnight at 4°C, followed by washing twice with PBS. These plates were used as CH-296-coated plates in the experiments described below.

### Example 8

### Culture of human PBMCs

Five samples of mononuclear cells (PBMCs) obtained from peripheral blood of five human donors by apheresis and stored in a frozen state (designated TK5, TK14, TK19, TK23 and TK29, respectively) were thawed and suspended in RPMI1640 medium (Sigma-Aldrich Japan K.K.). Each suspension was centrifuged at 500 x g for 10 minutes, the supernatants were removed, and cell pellets were obtained. The pellet was suspended in 10 mL of culture medium GT-T502 (Takara Bio Inc.) containing 1% autologous plasma, 0.2% human serum albumin (Baxter International Inc.), 600 IU/mL rhIL-2(CHIRON CORPORATION), and 2.5 µg/mL Fungizone (Bristol-Myers K.K.) to count the cells, and then adjusted to a cell density of 5 x 10⁶ cells/mL with the same medium. The culture was started by transferring 5 mL of each suspension to a 25cm² flask (Corning Incorporated), followed by addition of anti-CD3 antibody OKT-3 (Janssen Pharmaceutica N.V.) at a concentration of 30 ng/mL. On day 3 of the culture, the cells were counted and suspended in fresh culture medium at a cell density of 1-2 x 10⁶/mL to prepare a cell suspension.

### Example 9

### Gene transfer to human PBMCs

To each well of the CH-296-coated24-well and 12-well plates prepared in Example 7, 1 mL and 2 ml, respectively, of the recombinant retrovirus GaLV/MT-HLTR-MazF-PL solution prepared in Example 2 were added. The plates were then centrifuged at 32°C and 2000 x g for 2 hours to facilitate adhesion of the retroviral vector to RetroNectin. Each well of the 12-well plate was washed twice with 1 mL of PBS containing 1.5% human serum albumin, and filled with 1 mL of PBS containing 1.5% human serum albumin. The plate was stored at 4°Cfor the gene transfer to be carried out on the next day.

Each well of the 24-well plate was washed with 0.5 mL of PBS containing 1.5% human serum albumin, and 1 mL of the cell suspension prepared in Example 8 was added to each well. The plate was then centrifuged at 32°C and 1000 x g for 10 minutes to facilitate cell adhesion to the plate on which the retroviral vector was attached. After subsequent culture for 4 hours at 37°C in 5% carbon dioxide atmosphere, the culture was diluted with the culture medium to a cell density of 5 x 10⁵/mL and further cultured.

On the next day of the first gene transfer, the supernatant was removed from the stored 12-well plate, to which the virus adhered and which was filled with PBS containing 1.5% human serum albumin. To the wells of the plate, the total volume of each donor's transfected cells was transferred. The plate was centrifuged at 32°C and 1000 x g for 10 minutes to facilitate cell adhesion to the plate on which the retroviral vector was attached. After subsequent culture for 4 hours at 37°C in 5% carbon dioxide atmosphere, the culture was diluted with the culture medium to a cell density of 3 x 10⁵/mL and further cultured.

As a control, the same procedure was carried out on a CH-296 coated plate to which no retroviral vector was attached to prepare untrasfected cells.

After culturing for another three days from the second gene transfer, the cells were collected, stained for LNGFR-positive cells using anti-CD271-PE antibody, and analyzed by the flow cytometer Epics XL (Beckman Coulter Inc.) to determine the gene transfer efficiency. The gene transfer efficiencies to each donor-originated PBNC are shown in Table 2.

**Table 2**

| MazF gene transfer efficiency to each donor-derived PBMCs | |
|---|---|
| Donor | Gene transfer efficiency |
| TK5 | 69.6% |
| TK14 | 53.5% |
| TK19 | 54.9% |
| TK23 | 44.0% |
| TK29 | 47.5% |

### Example 10

### Experiment of HIV-infection to MazF-transfected PBMCs-1

The MazF-transfected cells originated from five donors and the untransfected cells prepared in Example 9 were respectively diluted with culture medium to a cell density of 5 x 10⁵/mL. Ten mL aliquot of the dilution was incubated overnight. On the next day, the cell suspension was divided into two aliquots, which were transferred into different centrifuge tubes. To one of the tubes, the HIV IIIB strain was added at MOI of 0.01. The other tube was mock inoculated with HIV-free culture medium. The tubes were incubated at 37°C for two hours to allow HIV IIIB enter the cells, which were then washed three times with GT-T503 medium and subsequently suspended in 3.5 mL of culture medium. One milliliter of this cell suspension was added to each well of a 24-well plate and incubated with 1 mL of culture medium for culture. This culture was carried out using three wells per sample. Three days after the infection, 1 mL of the culture supernatant was sampled, and 1 mL of culture medium was added to each of the wells and the culture was continued. Six days after the infection, 1 mL of the supernatant was sampled. The amount of HIV was determined by ELISA by quantifying the HIV p24 antigen present in the sampled supernatant. The cells after 6 days after the infection was subjected to MTT assay to determine the number of viable cells.

The results of the determination of viable cells are shown in Fig. 3(a)-(e), and of the quantification of p24 antigen on days 3 and 6 are shown in Table 3. In Fig. 3, the vertical axis represents the relative ratio (%) of viable cells in each test group to those in the control group (MazF-untransfected, HIV-uninfected). The horizontal axis of the graph represents the test groups. The numbers under the axis represent the donor numbers and the groups indicated with (-) correspond to the MazF-untransfected groups and the groups indicated with (+) correspond to the MazF-transfected groups; M denotes Moc infection (HIV-uninfection); and H denotes HIV-infection. There was no significant variation in viable cell count between the MazF-transfected and -untrasfected, or the HIV-infected and -uninfected groups in any donor PBMCs, showing that MazF, whose expression is induced by HIV-infection, does not induce cell death. As shown in Table 3, while the MazF gene transfer efficiencies to each donor cells were approximately 50%, the HIV replication was inhibited in the MazF-transfected cells to about 1/5 of the untransfected cells.

**Table 3**

| Results of quantification of HIV on days 3 and 6 post-infection | | | | | |
|---|---|---|---|---|---|
| Sample | | day 3 p24 (pg/mL) | Relative amount to uninfected cells on day 3 (%) | day 6 p24 (pg/mL) | Relative amount to uninfected cells on day 6 (%) |
| TK5 | Untrasfected | 2700.3 | | 11065.7 | |
| | MazF-transfected | 366.0 | 13.6 | 2213.0 | 20.0 |
| TK14 | Untrasfected | 3823.0 | | 23451.7 | |
| | MazF-transfected | 914.0 | 23.9 | 4881.7 | 20.8 |
| TK19 | Untrasfected | 4365.7 | | 338276.7 | |
| | MazF-transfected | 843.7 | 19.3 | 53453.0 | 15.8 |
| TK23 | Untrasfected | 509.0 | | 38827.3 | |
| | MazF-transfected | 151.3 | 29.7 | 8492.0 | 21.9 |
| TK29 | Untrasfected | 1099.3 | | 28104.7 | |
| | MazF-transfected | 164.0 | 14.9 | 6168.7 | 21.9 |

### Example 11

### Experiment of HIV-infection to MazF-transfected PBMCs-2

MazF-transfected and untransfected cells were prepared from PBMCs of TK19 origin according to the same procedure as that in Example 9. The gene transfer efficiency of the transfected cells thus prepared was 61.1%. To each of these cells, the HIV IIIB and HIV HE strains were added and infected at MOI of 0.01 according to the same method as that described in Example 10. As a control, Moc infection was prepared by addition of the HIV-free culture medium. The amount of HIV present in the supernatant was determined by quantifying the HIV p24 antigen present in the supernatant by ELISA 3 and 6 days after the infection. The results of the quantification of p24 antigen on days 3 and 6 are shown in Table 4.

As shown in Table 4, while the MazF gene transfer efficiency in MazF-transfected cells was 61.1%, the HIV replication of the HIV IIIB and HIV HE strains was inhibited to about 1/20 and 1/4, respectively, as compared with the untransfected cells.

**Table 4**

| Sample | | day 3 p24 (pg/mL) | Relative amount to uninfected cells on day 3 (%) | day 6 p24 (pg/mL) | Relative amount to uninfected cells on day 6 (%) |
|---|---|---|---|---|---|
| TK19/ IIB infection | Untrasfected | 123.7 | | 7846.3 | |
| | MazF-transfected | 0 | 0 | 393.7 | 5.0 |
| TK14/ HE infection | Untrasfected | 383.3 | | 7495.3 | |
| | MazF-transfected | 162.0 | 42.3 | 2128.0 | 28.4 |

### Industrial Applicability

The present invention provides a retroviral vector useful for the treatment and/or prevention of cancers and virus infections. Since cells introduced with the above nucleic acid construct are capable of inhibiting replication of RNA-viruses, it is particularly effective for the treatment and/or prevention of diseases caused by such viruses, for example, HIV-infection.

### Sequence Listing Free Text

SEQ ID NO:2; Synthetic DNA encoding MazF.
SEQ ID NO:3; Primer to amplify HIV-LTR-MazF cassette.
SEQ ID NO:4; Primer to amplify HIV-LTR-MazF cassette.
SEQ ID NO:5; Primer to amplify pA fragment.
SEQ ID NO:6; Primer to amplify pA fragment.
SEQ ID NO:7; Primer to amplify pPGK fragment.
SEQ ID NO:8; Primer to amplify pPGK fragment.
SEQ ID NO:9; Primer to amplify LNGFR fragment.
SEQ ID NO:10; Primer to amplify LNGFR fragment.

## Claims

1. A retroviral vector which comprises a transcription unit comprising a transcription regulatory sequence and a gene encoding a polypeptide having single-stranded RNA-specific endoribonuclease activity which is placed so that its expression can be controlled by the regulatory sequence, wherein the unit is placed so that the direction of transcription of mRNA from the unit is opposite to the direction of transcription of RNA genome of the retroviral vector.

2. The retroviral vector according to claim 1, wherein the transcription regulatory sequence is a sequence for controlling the transcription of a gene expressed specifically in cancer cells or virus-infected cells.

3. The retroviral vector according to claim 1, wherein the transcription regulatory sequence is such a sequence that the transcription is induced by a trans-acting factor of a cancer cell or virus origin.

4. The retroviral vector according to claim 3, wherein the transcription regulatory sequence is such a sequence that the transcription is induced by a trans-acting factor of an immunodeficiency virus.

5. The retroviral vector according to claim 4, which comprises such a transcription regulatory sequence that the transcription is induced by Tat protein and/or Rev protein.

6. The retroviral vector according to claim 4, wherein a gene encoding a polypeptide having single-stranded RNA-specific endoribonuclease activity is placed downstream of the LTR of an immunodeficiency virus.

7. The retroviral vector according to claim 1, wherein the transcription regulatory sequence is such a sequence that the transcription is induced by a trans-acting factor artificially supplied into cells.

8. The retroviral vector according to any one of claims 1 to 7, wherein the polypeptide having single-stranded RNA-specific endoribonuclease activity is MazF protein.

9. The retroviral vector according to any one of claims 1 to 8 for use in a method for treating or preventing a disease.

10. The retroviral vector for use in a method for treating or preventing a disease according to claim 9, wherein the disease is a cancer or a virus infection.

11. The retroviral vector for use in a method for treating or preventing a disease according to claim 9, wherein the disease is an immunodeficiency virus infection.

12. A therapeutic or preventive agent of a disease, which comprises the retroviral vector according to any one of claims 1 to 8 as an effective component.

13. The therapeutic or preventive agent according to claim 12 for use in a method of treating or preventing cancer or virus infection.

14. The therapeutic or preventive agent according to claim 13 for use in a method of treating or preventing an immunodeficiency virus infection.

## Patentansprüche

1. Retroviraler Vektor, der eine Transkriptionseinheit umfasst, die eine transkriptionsregulatorische Sequenz und ein Gen umfasst, das für ein Polypeptid mit einer für einzelsträngige RNA spezifischen Endoribonuclease-Aktivität kodiert und das derart platziert ist, dass seine Expression durch die regulatorische Sequenz kontrolliert werden kann, wobei die Einheit derart platziert ist, dass die Richtung einer Transkription von mRNA von der Einheit entgegengesetzt zu der Richtung einer Transkription eines RNA-Genoms des retroviralen Vektors ist.

2. Retroviraler Vektor gemäß Anspruch 1, wobei die transkriptionsregulatorische Sequenz eine Sequenz zum Kontrollieren der Transkription eines Gens ist, das spezifisch in Krebszellen oder virusinfizierten Zellen exprimiert wird.

3. Retroviraler Vektor gemäß Anspruch 1, wobei die transkriptionsregulatorische Sequenz eine solche Sequenz ist, so dass die Transkription durch einen trans-wirkenden Faktor einer Krebszelle oder viralen Ursprungs induziert wird.

4. Retroviraler Vektor gemäß Anspruch 3, wobei die transkriptionsregulatorische Sequenz eine solche Sequenz ist, so dass die Transkription durch einen trans-wirkenden Faktor eines Immundefizienz-Virus induziert wird.

5. Retroviraler Vektor gemäß Anspruch 4, der eine solche transkriptionsregulatorische Sequenz umfasst, so dass die Transkription durch das Tat-Protein und/oder Rev-Protein induziert wird.

6. Retroviraler Vektor gemäß Anspruch 4, wobei ein Gen, das für ein Polypeptid mit einer für einzelsträngige RNA spezifischen Endoribonuclease-Aktivität kodiert, stromabwärts des LTR eines Immundefizienz-Virus platziert ist.

7. Retroviraler Vektor gemäß Anspruch 1, wobei die transkriptionsregulatorische Sequenz eine solche Sequenz ist, so dass die Transkription durch einen trans-wirkenden Faktor, der künstlich in Zellen bereitgestellt wird, induziert wird.

8. Retroviraler Vektor gemäß einem jeglichen der Ansprüche 1 bis 7, wobei das Polypeptid mit einer für einzelsträngige RNA spezifischen Endoribonuclease-Aktivität MazF-Protein ist.

9. Retroviraler Vektor gemäß einem jeglichen der Ansprüche 1 bis 8 für eine Verwendung in einem Verfahren zum Behandeln oder Verhindern einer Erkrankung.

10. Retroviraler Vektor für eine Verwendung in einem Verfahren zum Behandeln oder Verhindern einer Erkrankung gemäß Anspruch 9, wobei die Erkrankung Krebs oder eine Virusinfektion ist.

11. Retroviraler Vektor für eine Verwendung in einem Verfahren zum Behandeln oder Verhindern einer Erkrankung gemäß Anspruch 9, wobei die Erkrankung eine Infektion mit einem Immundefizienz-Virus ist.

12. Therapeutisches oder präventives Mittel einer Erkrankung, das den retroviralen Vektor gemäß einem jeglichen der Ansprüche 1 bis 8 als eine wirksame Komponente umfasst.

13. Therapeutisches oder präventives Mittel gemäß Anspruch 12 für eine Verwendung in einem Verfahren zum Behandeln oder Verhindern von Krebs oder einer Virusinfektion.

14. Therapeutisches oder präventives Mittel gemäß Anspruch 13 für eine Verwendung in einem Verfahren zum Behandeln oder Verhindern einer Infektion mit einem Immundefizienz-Virus.

## Revendications

1. Vecteur rétroviral qui comprend une unité de transcription comprenant une séquence régulatrice de transcription et un gène codant pour un polypeptide ayant une activité d'endoribonucléase spécifique pour un ARN monobrin, qui est placé de telle manière que son expression peut être commandée par la séquence régulatrice, tandis que l'unité est placée de telle manière que la direction de transcription de l'ARNm à partir de l'unité est opposée à la direction de transcription du génome d'ARN du vecteur rétroviral.

2. Vecteur rétroviral selon la revendication 1, dans lequel la séquence régulatrice de transcription est une séquence pour commander la transcription d'un gène exprimé spécifiquement dans des cellules cancéreuses ou des cellules infectées par un virus.

3. Vecteur rétroviral selon la revendication 1, dans lequel la séquence régulatrice de transcription est une séquence telle que la transcription est induite par un facteur trans-agissant ayant son origine dans une cellule cancéreuse ou un virus.

4. Vecteur rétroviral selon la revendication 3, dans lequel la séquence régulatrice de transcription est une séquence telle que la transcription est induite par un facteur trans-agissant d'un virus d'immunodéficience.

5. Vecteur rétroviral selon la revendication 4, qui comprend une séquence régulatrice de transcription telle que la transcription est induite par une protéine Tat et/ou une protéine Rev.

6. Vecteur rétroviral selon la revendication 4, dans lequel un gène codant pour un polypeptide ayant une activité d'endoribonucléase spécifique pour un ARN monobrin est placé en aval de la LTR d'un virus d'immunodéficience.

7. Vecteur rétroviral selon la revendication 1, dans lequel la séquence régulatrice de transcription est une séquence telle que la transcription est induite par un facteur trans-agissant artificiellement procuré dans des cellules.

8. Vecteur rétroviral selon l'une quelconque des revendications 1 à 7, dans lequel le polypeptide ayant l'activité d'endoribonucléase spécifique pour un ARN monobrin est la protéine MazF.

9. Vecteur rétroviral selon l'une quelconque des revendications 1 à 8 pour utilisation dans un procédé pour le traitement ou la prévention d'une maladie.

10. Vecteur rétroviral pour utilisation dans un procédé pour le traitement ou la prévention d'une maladie selon la revendication 9, dans lequel la maladie est un cancer ou une infection virale.

11. Vecteur rétroviral pour utilisation dans un procédé pour le traitement ou la prévention d'une maladie selon la revendication 9, dans lequel la maladie est une infection par un virus d'immunodéficience.

12. Agent thérapeutique ou préventif d'une maladie, qui comprend le vecteur rétroviral selon l'une quelconque des revendications 1 à 8 à titre de composant efficace.

13. Agent thérapeutique ou préventif selon la revendication 12, pour utilisation dans un procédé pour le traitement ou la prévention de cancer ou d'infection virale.

14. Agent thérapeutique ou préventif selon la revendication 13, pour utilisation dans un procédé pour le traitement ou la prévention d'une infection par un virus d'immunodéficience.


### SEQUENCE LISTING

<110> TAKARA BIO INC.
<120> Vector for gene therapy
<130> 668252
<150> JP 2007-112178
   <151> 2007-04-20
<150> JP 2007-138889
   <151> 2007-05-25
<150> JP 2007-268310
   <151> 2007-10-15
<150> JP 2008-025248
   <151> 2008-02-05
<160> 12
<170> Patent In version 3. 3
<210> 1
   <211> 111
   <212> PRT
   <213> Escherichia coli
<400> 1
   Met Val Ser Arg Tyr Val Pro Asp Met Gly Asp Leu Ile Trp Val Asp
   1               5                   10                  15
   Phe Asp Pro Thr Lys Gly Ser Glu Gln Ala Gly His Arg Pro Ala Val
               20                  25                  30
   Val Leu Ser Pro Phe Met Tyr Asn Asn Lys Thr Gly Met Cys Leu Cys
           35                  40                  45
   Val Pro Cys Thr Thr Gln Ser Lys Gly Tyr Pro Phe Glu Val Val Leu
       50                  55                  60
   Ser Gly Gln Glu Arg Asp Gly Val Ala Leu Ala Asp Gln Val Lys Ser
   65                  70                  75                  80
   Ile Ala Trp Arg Ala Arg Gly Ala Thr Lys Lys Gly Thr Val Ala Pro
                   85                  90                  95
   Glu Glu Leu Gln Leu Ile Lys Ala Lys Ile Asn Val Leu Ile Gly
               100                 105                 110
<210> 2
   <211> 336
   <212> DNA
   <213> Artificial
<220>
   <223> Synthetic DNA encodi ng MazF.
<400> 2
   at ggt aagcc gat acgt acc cgat at gggc gat ct gat t t gggt t gat t t t gacccgacc 60
   aaaggt agcg agcaagct gg ccat cgt cca gct gt t gt cc t gagt cct t t cat gt at aat 120
   aat aaaaccg gt at gt gt ct gt gt gt t cct t gt accacgc aat caaaagg at at ccgt t c 180
   gaagt t gt t t t at ccggt ca ggaacgt gat ggcgt agcgt t agct gat ca ggt aaaaagt 240
   atcgcctggc gggcaagagg agcaacgaag aaaggaaccg ttgccccaga ggaactgcag   300
   ct cat t aaag ccaaaat t aa cgt act gat t ggt t aa 336
<210> 3
   <211> 26
   <212> DNA
   <213> Artificial
<220>
   <223> Primer t o amplify HIV- LTR- MazF cassette.
<400> 3
   cggccgct gg aagggct aat t t ggt c 26
<210> 4
   <211> 34
   <212> DNA
   <213> Artificial
<220>
   <223> Primer t o amplify HI V- LTR- MazF cassette.
<400> 4
   gcggccgct t aaccaat cag t acgt t aat t t t gg 34
<210> 5
   <211> 29
   <212> DNA
   <213> Artificial
<220>
   <223> Primer to amplify pA fragment.
<400> 5
   gcggccgcgg gggaggctaa ctgaaacac 29
<210> 6
   <211> 26
   <212> DNA
   <213> Artificial
<220>
   <223> Primer to amplify pA fragment.
<400> 6
   acgcgtggct atggcagggc ctgccg 26
<210> 7
   <211> 39
   <212> DNA
   <213> Artificial
<220>
   <223> Primer t o amplify pPGK fragment.
<400> 7
   acgcgt gcgg ccgcagat ct aattctaccg ggt agggga 39
<210> 8
   <211> 39
   <212> DNA
   <213> Artificial
<220>
   <223> Primer to amplify pPGK fragment.
<400> 8
   ct cgaggcgg cgcggat ccc t gcaggt cga aaggcccgg 39
<210> 9
   <211> 34
   <212> DNA
   <213> Artificial
<220>
   <223> Primer t o amplify LNGFR fragment.
<400> 9
   gcgct gat ca gaat t cgccg cggccagct c cggc 34
<210> 10
   <211> 31
   <212> DNA
   <213> Artificial
<220>
   <223> Primer t o amplify LNGFR fragment.
<400> 10
   gcgcct cgag t ct agaggat ccccct gt t c c 31
<210> 11
   <211> 82
   <212> PRT
   <213> Escher i chi a coli i
<400> 11
   Met Ile His Ser Ser Val Lys Arg Trp Gly Asn Ser Pro Ala Val Arg
   1 5 10 15
   Ile Pro Ala Thr Leu Met Gln Ala Leu Asn Leu Asn Ile Asp Asp Glu
   20 25 30
   Val Lys Ile Asp Leu Val Asp Gly Lys Leu Ile Ile Glu Pro Val Arg
   35 40 45
   Lys Glu Pro Val Phe Thr Leu Ala Glu Leu Val Asn Asp Ile Thr Pro
   50 55 60
   Glu Asn Leu His Glu Asn Ile Asp Trp Gly Glu Pro Lys Asp Lys Glu
   65 70 75 80
   Val Trp
<210> 12
   <211> 249
   <212> DNA
   <213> Escheri chi a col i
<400> 12
   atgatccaca gtagcgtaaa gcgttgggga aattcaccgg cggtgcggat cccggctacg 60
   ttaatgcagg cgctcaatct gaatattgat gatgaagtga agattgacct ggtggatggc 120
   aaattaatta ttgagccagt gcgtaaagag cccgtattta cgcttgctga actggtcaac 180
   gacatcacgc cggaaaacct ccacgagaat atcgactggg gagagccgaa agataaggaa 240
   gtctggtaa 249